# EUROPEAN PATENT APPLICATION

(11) **EP 4 183 864 A1**
(43) Date of publication of application: **24.05.2023**
(21) Application number: 22208948.4
(22) Date of filing: 22.11.2022
(51) Int. Cl.: C12M 1/00, C12M 1/34

(54) **PROCESS AND DEVICES FOR EFFICIENT PARTIAL OR TOTAL SOLID-LIQUID SEPARATION USING GAS-CONTROLLED CONDITIONS**

(30) Priority: 22.11.2021 US 202163282083 P
(71) Applicant: Mycofood US LLC, London SE6 2AR (GB)
(72) Inventor: Blasco, Martin, Buenos Aires (AR); Acerbo, Horacio Claudio, Buenos Aires (AR)
(74) Representative: Padial Martinez, Ana Belen

(57) **Abstract**

A filtration system for downstream processing of a suspension containing a labile solid material in a liquid medium, wherein dissolved gas in liquid environment is controlled to ensure optimal process conditions. The dissolved gas can be controlled by a gas-liquid interface by bubbling; a liquid-liquid interphase transfer process using a gas vector substance; a liquid-solid interphase transfer process using a gas permeable solid or a reaction evolving oxygen or other gas/gases. The retained solid material is removed in batch operations, continuous operations or mixed-type operations. The process can also include further filtration, heat-treatment, and removal of at least a part of the liquid. The filtration system could also process a concentrated suspension which is devoid of the free liquid, then heat-treated and the liquid produced by the treatment removed by further filtration. The solid material can be a biomass.

## Description

### Cross Reference to Related Applications

This application claims priority to U.S. Provisional Patent Application No. 63/282,083, filed November 22, 2021, the contents of which are incorporated by reference in their entirety.

### Technical Field of the Invention

The invention refers to the technical field of processes and devices to obtain partial or total separation of a labile solid from a liquid in a production process. In particular, the invention uses gas-controlled conditions for the production of biomass and its efficient separation from the dispersant liquid. The invention is particularly useful in processes to reduce RNA content of a biomass.

### Background of the Invention

Filtration is the most common process in industrial operations for dispersed solid-liquid separations. Processes for separation of inert materials are well studied and parameterized.

However, in the context of the processing substances sensitive to dissolved gases (reactive) or involving (micro- and macro-) organisms requiring gas components for proper development, conditions during filtration can affect the desired final composition. For example, in the presence of excess sugar or under poor oxygen conditions, processes utilizing bio- active material can result in the formation of metabolites or reaction by-products, or the metabolism pathways of the biomaterial can change, reducing the yield or quality of the end-product from the process. Further, the accumulation of gases produced during the reaction and subsequent separation processes can affect the process conditions and result in yield reduction or loss of valuable products.

A key step in biomass production processes (for example from bacteria, yeasts, filamentous fungi, etc.) is reduction of RNA content in the biomass downstream of the bioreactor and before the preparation of a final manufactured product, especially in the case where the biomass is a fungal biomass (either from a filamentous fungus or yeasts) and other biochemically active products with high RNA content. Existing processes are known and well characterized (See, for example, UK Patent Application Nos.GB2557886 and GB2551964, and International Publication Nos. WO2018/002581 and WO2018/002579)

At present, the methods to reduce biomass RNA content involve a two-step process with two heating treatments, one immediately after the other. For example, WO2018002579 describes a process in which a first step includes the heating from 40 to 69 °C, and a second step of increasing the temperature by 2 to 20 °C more, after which the biomass is separated from the other components. The treatment machinery is arranged to use the hot material from the second step as a heat source to increase the temperature in the inflow of the first step.

For biomass producing facilities, the treatment of huge amounts of whole liquid stream (grown microorganisms in a dispersant liquid) containing mostly water is an energy intensive process. The filtration of the material is a viable approach to reduce the liquid content allowing the treatment of the solid material. Unfortunately, filtration processes generally occur in an environment where the gas component in the liquid cannot be controlled. Under these conditions, changes are observed in the products that reduce the yield and result in undesirable by-products. Furthermore, temperature control (i.e. heating) of systems displaying complicated topologies and moving parts is performed by contacting the material to be heated with high temperature fluids or surfaces, which can lead to degradation resulting in lower yields and/or undesirable by-products.

### Summary of the Invention

The present invention solves these and other problems in the prior art by providing a process where the dissolved gases concentration is controlled to a desired level using solid-liquid, liquid-liquid or gas-liquid interfaces, ensuring the control of the dissolved gas level in the liquid, and thus controlling the reaction environment while the processes inside the filter system are occurring. The use of microwaves as a heating means can also allow heat treatment of the material within the device without the need of using steam or other hot fluid or convective wall transfer. The present invention provides a significant improvement to processes and systems used for separation purposes and solves the main issues generally observed in current industrial separation operations.

The present invention is a filtration method for downstream processing of a suspension containing a labile solid material in a liquid medium that includes controlling the dissolved gas content in the liquid during filtering to optimize process conditions. The dissolved gas content can be controlled by a gas-liquid interphase transfer process by bubbling gas with a composition and a rate to provide a desired gas content; a liquid-liquid interphase transfer process using a gas vector substance charged with a concentration of gas to provide a desired gas content; a liquid-solid interphase transfer process by using a gas permeable solid having a concentration and pressure of gas by the circulation of gas or a gas vector substance within a luminal structure to provide a desired gas content; or controlling the dissolved gas content by use of a reaction evolving oxygen or other gas/gases.

The solid material is removed in a batch operation or a continuous operation. The solid material can also be removed in a mixed-type operation.

Optionally, in any of the above embodiments, the invention can also include concentrating the suspension by filtration, heat-treatment, and removal of at least a part of the liquid filtrate from the filtration. In embodiments, removal of the filtrate is followed by heat-treatment and subsequent removal of additional liquid produced by the heat-treatment by further filtration. Heat treatment can be accomplished using steam, a heated gas, a heated liquid, or combinations thereof. In some embodiments, the heat treatment is accomplished by treatment with microwaves.

In any of the above embodiments, the solid material is a biomass In embodiments, the heat treatment inactivates the biomass. The biomass may be a fungus, for example a yeast or a filamentous fungus; cells of animal origin; cells of plant or algal origin; cells of protist origin; or a virus or part thereof. The solid material can also be a mixture of two or more of a fungus, cells of animal origin, cells of plant origin, and cells of algal origin. The solid material can be in the form of, for example, a colloid or an emulsified solid or a mixture thereof.

### Brief Description of the Drawings

The foregoing and other features and advantages of the invention will be apparent from the following, more particular description of a preferred embodiment of the invention, as illustrated in the accompanying drawings wherein like reference numbers generally indicate identical, functionally similar, and/or structurally similar elements.
Figure 1 is a flow diagram of an exemplary process according to the invention for RNA reduction and biomass processing using a centrifugal filtration device.
Figure 2 is a schematic diagram of an exemplary system for RNA reduction and biomass processing using a centrifugal filtration device.
Figure 3 is a schematic diagram of an exemplary system for RNA reduction and biomass processing configuration using a tangential filtration device with gas diffusion component for oxygen control within the filter.
Figure 4 is a schematic diagram of an exemplary system for RNA reduction and biomass processing configuration using a DSM type filter with gas composition control for oxygen control within the filter.

### Detailed description of the invention

The invention is focused on the field of processes and devices for processing reactive entities susceptible to environment conditions during filtration processes. The reactive entity is a solid material that is suspended in a liquid. The solid is a biomass such as a bacteria, a yeast, or a type of fungi produced by fermentation or other process known in the art. In embodiments, the fungus is a filamentous fungus. The devices and methods of the invention are used for separation operations where diffusive or convective transport allows for a reactive entity to survive the process unchanged or with a desired change, avoiding difficulties arising from steps generally performed in separation equipment. The unchanged or changed state refers to the desired condition for the entity in the context of the process. The temperature within the device is controlled to a desired level in various ways, being preferred but not limited to the use of microwaves to heat water and material susceptible to microwave heating. Use of microwave heating is advantageous in avoiding temperature gradients caused by wall-to-processed-material convection.

The process of the invention includes production enhancement of the reactive entity, separation, and content transformation to provide a desired composition, wherein the inactivation is accomplished while maintaining stability of the reactive entity. Also, the process provides the production of soluble or dispersive final product from the reactive entity. While systems and methods of the invention may apply to a broad range of reactive entities, the invention is particularly used in the production of biomass, particularly for biomass that is used to produce food products. In particular examples, the biomass is a filamentous fungus and the invention is used for removal of RNA and byproducts from the biomass.

The present invention refers to a process where dissolved gas concentration is controlled using solid-liquid, liquid-liquid or gas-liquid interfaces, ensuring the control of dissolved gas level in the liquid during manufacture (for example by fermentation), and controlling the reaction environment during filtration processes. Furthermore, the use of microwaves as a heating means allows heat treatment of the material within the device without the need to use steam or any other hot fluid or convective wall transfer.

Biomass production is generally performed in bioreactors (stirred tanks, airlift, bubble column, staked trays, perfusion) to grow a microorganism aerobically. For example, according to the production conditions for fungal biomass, in the particular case of *Fusarium venenatum,* the culture conditions are around 28 °C and pH 5.8 with limited control of concentration of carbon, energy source (i.e. glucose, sucrose, starch) and nitrogen source (i.e. ammonium hydroxide, urea, amino acids). Bioreactors work by controlling the pH and temperature in order to keep both the growth rate and the composition of the biomass maximized. Nutrients are added at a rate compatible with the productivity requirements for the industrial processing, and mechanical conditions are adjusted to ensure the appropriate shear stress to keep a high percentage of viable cells and maintain a proper physical structure for product applications. There are a number of configurations able to produce the biomass including, for example, submerged culture (SmF), solid state culture (SSF) and mixed culture strategies (SmF+SSF). Any of these can be followed by a processing step involving RNA reduction. In the case of SmF, the processing is generally performed with a liquid accompanying the biomass, whereas in the case of SSF it is generally required to add liquid in order to eliminate soluble molecules secreted by the biomass upon heat treatment. The present invention can be used for any biomass as it maximizes concentration of supernatant, increases the recovery of biomass and reduces energy costs to obtain the biomass and the by-products.

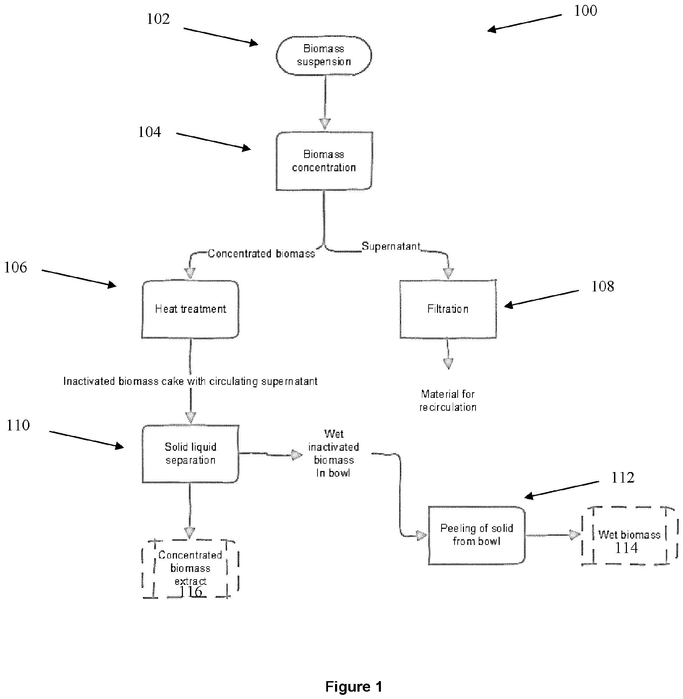

In view of the above, Figure 1 illustrates a flow chart of an exemplary embodiment of the invention that may be used for the separation of RNA from a biomass. As will be appreciated by persons skilled in the art, the process of Figure 1 is readily modified to provide improved separations in other situations, for example from suspensions containing labile solids other than biomass or for separating different by-products. Furthermore, as described further herein, there may be additional steps in the process, and some materials may be recirculated for additional purposes or additional recovery. Specific and alternative apparatuses and methodologies are described elsewhere herein.

Figure 1 illustrates a process **100** for isolation of a solid biomass from a liquid suspension. According to this exemplary embodiment, in step **102** a biomass suspension is provided, for example from a fermenter. The biomass is then concentrated in step **104** using a filtering device, for example a filter or centrifuge, as described elsewhere herein, to provide predominately a solid concentrated biomass and predominantly a liquid supernatant that contains culture media, including waste products and by-products, and may contain additional suspended biomass. During the concentrating step **104,** the gas composition of the liquid is controlled to provide an environment similar to that used in the fermentation phase. It has surprisingly been found that controlling gas concentration during this stage has a beneficial effect on the yield and purity of the final product. The supernatant may go through further processing or treatment, including, for example an additional filtration step **108,** as described elsewhere herein. The supernatant may also be stored in which case temperature and gas composition may be controlled to avoid oxidation or degradation. Preservatives may also be added to avoid contamination by ambient microbiota.

The concentrated biomass can undergo further treatment, which may include heat treatment 106 and/or oxygen reduction to inactivate the biomass. Heat treatment may utilize the supernatant for heating. In some embodiments, the supernatant is heated and then recirculated through the concentrated biomass, both inactivating the biomass and providing a means for isolating additional biomass from the supernatant in an additional solid-liquid separation step 110. In some embodiments, the supernatant is heated to about 50 to 75 °C. In the case of perfusion bioreactors the temperature can be lowered to be more compatible to mammalian cell culture (37 °C). Also during this step, any solids suspended in the supernatant may be returned to the main separation device where the presence of the initial biomass acts as an extra filtration substrate improving filtration and the amount of material accumulated in the filtration apparatus. Particularly in the case where the additional solid-liquid separation step **110** involves centrifugation, rather than heating the supernatant, the material in the filtering device, for example the concentrated biomass and the recycled supernatant, may be heated using microwave energy. This additional separation provides a wet inactivated **114** biomass which, for example, may be on a filter device or in the bowl of a centrifuge, which is then removed in step **112** to provide a wet biomass. The wet biomass **114** then undergoes further processing to form a final product. The additional solid-liquid separation step **110** also results in the isolation of a concentrated biomass extract **116.**

The concentrated biomass **116** extract may undergo further processing such as isolating RNA monomers and oligomers therefrom or recirculation and regeneration to provide new culture media. The concentrated biomass extract may also be spray dried to produce a powder.

The wet biomass **114** can be spray dried for some applications but can also be dried differently, for example by convective or microwave heating using a tunnel drier. The solid composition can also be processed to control its physical characteristics. For example, oxygen contact with the solid composition can provide a stringent texture development due to oxidation on the surface of the solid. As steam controls temperature the properties of the biomass can be modified. A liquid buffer allowing removal or retention of particular components at the biomass would render a different biomass composition. Using a particular pH or salt can control the nature and amounts of proteins, amino acids, etc. that will be removed or retained during further processing.

Processes according to the invention can use various means for separation of solid from liquid, generally referred to here as filtration elements. Examples of suitable filtration elements include, but are not limited to:
1. Sheet or sheets of filtering material arranged in cassettes or other encasings.
2. Wedge wire filters.
3. Perfect pore filters.
4. Wire mesh filters.
5. Hollow fibers.
6. Deep filtration systems.
7. Continuous filtering devices (centrifuges and others)
and the like.

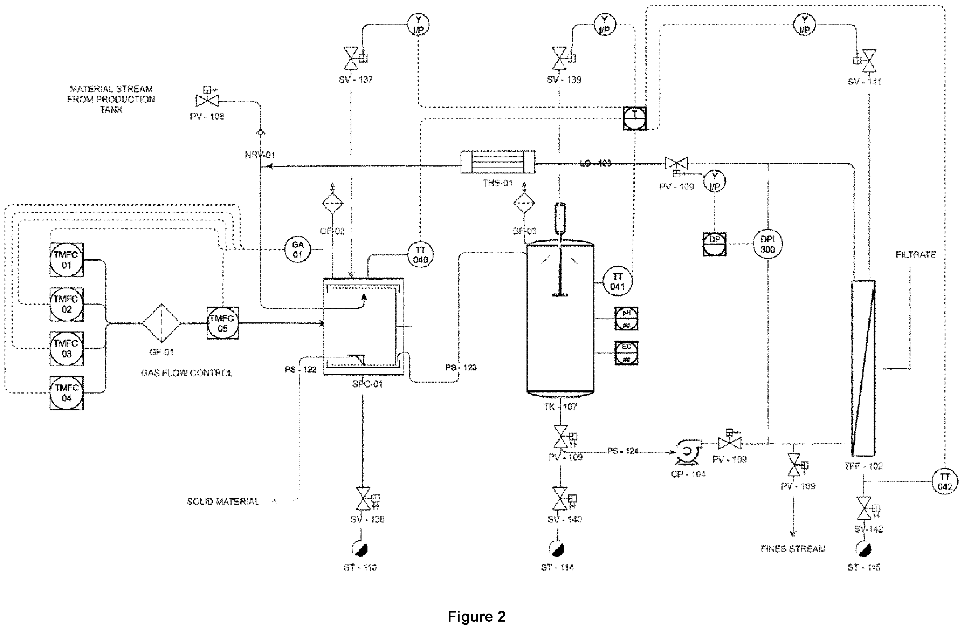

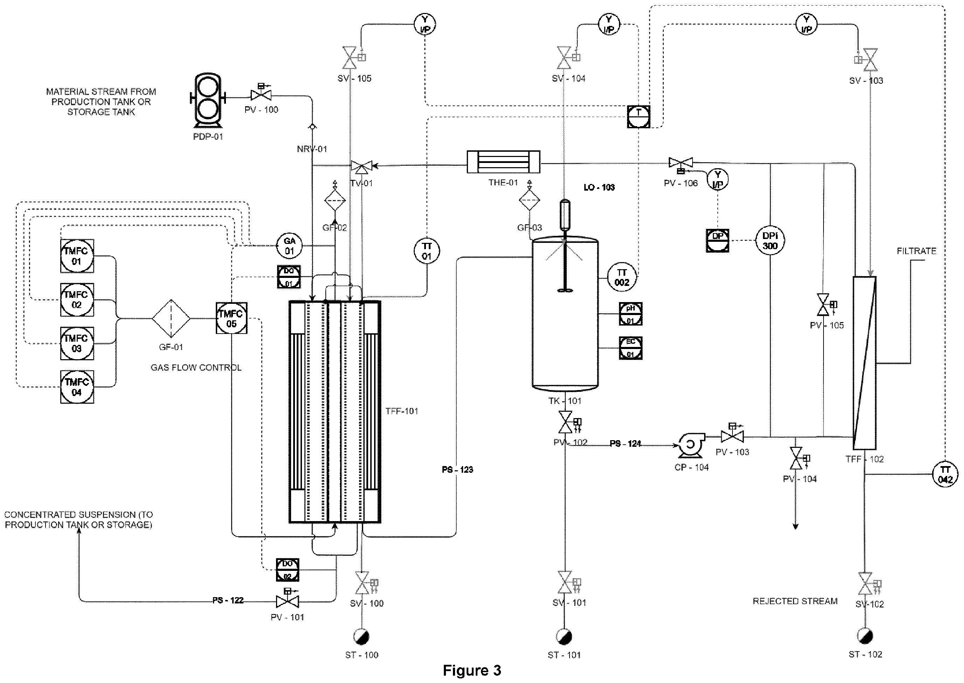

The filtration systems used for the process (with filtration elements 1 to 4) can be either stationary as in Nutsche filtration apparatus or moving as in rotary press filters, spin filter or rotary drums. Pusher centrifuges, peeler centrifuges, decanter centrifuges, etc. are also useful filtration mechanisms for the concentration of solids such as a biomass. In the examples that follow, biomass concentration utilizes a centrifuge (for example SPC-01 in Fig 2) or a filtration device (TFF-101 in Fig. 3 or DSM Filter in Fig. 2).

The level of the control of gas concentration during the solid concentration stage is based on the biochemical activity or stability of the material. For example, in the case of high respiratory activity, the composition can be oxygen rich composition, up to 100%, more commonly 20%. In later stages after the biomass is inactivated by heat treatment the oxygen content can be lowered to avoid undesired oxidation, in some cases lowering the concentration of O₂ to 0%.

The systems and methods of the invention control gas content in the associated liquids during production (e.g., fermentation) and filtering processes. It has been found that controlling the gas composition allows the production/control of particular physical and chemical properties in the material such as producing or avoiding oxidation, precluding the use of aggressive agitation resulting in a lower shear of the product which can lead to undesired textures in the material. Controlling gas composition can also reduce the presence of by-products and prevent changes in metabolism

There a number of strategies for gas infusion/diffusion control, particularly during the filtration process, such as:
1. Gas-liquid transfer (for example, nitrogen, oxygen, or air infusion directly into the liquid).
2. Liquid-liquid transfer (for example, using oxygen vectors such as perfluorodecalin)
3. Solid-liquid transfer (for example, diffusion of pressurized gas such as oxygen through a silicone hose)
4. Gas evolving reactions (for example, generating oxygen from peroxide decomposition, optionally in the presence of a catalyst).

The separation of labile solids from a liquid in which it is suspended, particularly with the use of filtering sleeves or tangential flow filtration apparatuses, is affected by the CO₂ built up and O₂ reduction in the filtering device and the change in temperature which can impact cell productivity. The control of the gas composition and temperature within the filtering devices with gas phase or gas vector control strategies as those developed in this invention improves the performance of perfusion cultures outperforming currently available perfusion by reducing CO₂ build up and increasing O₂ concentrations. The media acting as gas vectors can be adjusted in order to work in a range of conditions from the saturation to the total exhaustion of that gas component.

### Examples

The invention is described by reference to the following non-limiting examples. A person skilled in the relevant art will recognize that other components and configurations can be used without parting from the spirit and scope of the invention. The invention may be applied to other processes and functionally similar, and/or structurally similar elements may be substituted for the specific elements described.

The RNA reduction process is generally a temperature-controlled process, where the internal RNAses (endogenous RNA degrading enzymes) digest the RNA to its monomers reducing the RNA content within a biomass such as a fungus (particularly the mycelium of a filamentous fungus), bacteria, algae, etc. The process also results in increasing concentration of monomers and oligomers in the supernatant liquid.

In exemplary embodiments, the concentrated biomass extract is processed to provide a concentration of dissolved solids of more than 20% in order to allow cost-effective recovery of the dissolved material as dried solids using i.e. a spray dryer. Energy expenditure resulting from the treatment of excess liquid, and water evaporation is radically changed when liquid mass in contact with the RNA containing material is reduced using an appropriate filtering device.

### Example 1a. Application in RNA reduction using a centrifugal filtration device

In this example illustrated in Fig. 2, the use of a modified device consisting of a peeler syphon centrifuge with gas and temperature control is provided and shows the equipment for processing fungal biomass suspension. The equipment allows partial dewatering in the peeler syphon centrifuge (SPC-01), supernatant recirculation, heat treatment and final dewatering of the biomass. The gas composition and temperature in the SPC-01 is controlled to ensure the conditions to enhance productivity and accomplish RNA reduction and final water content of the biomass.

The bioreactor liquid stream is introduced into the filtration device (SPC-01) and the free liquid is partially removed to obtain a concentrated biomass with controlled oxygen level, allowing aerobic conditions. Once the desired biomass concentration is obtained, the material is heated to reach the RNA degradation temperature, for example a temperature from about 50 to about 70 °C, inactivating the biomass. Once the concentrated broth reaches the degradation temperature, the liquid is recirculated in the filtration system to ensure proper extraction of soluble matter and once the soluble concentration remains unchanged or reaches the desired composition, the liquid is separated and the solid removed from the filtration system. The heating process is produced by a microwave system ensuring the whole mass heating without fully depending on convective heat transfer as in other systems.

Briefly, the biomass suspended in the culture medium enters the SPC-01 (syphon peeler centrifuge) and liquid material is transferred to tank TK-107 for filtration and recirculation. The filtration allows the small size biomass recovery by filtration with the TFF-102 filter, recirculating the biomass to the SPC-01 and allowing the better recovery once a bed of material is formed within the centrifuge basket. The recirculation involves the passage through a heat exchanger THE-01 to adjust the temperature of the recirculating liquid to ensure proper temperature during heat treatment of the biomass retained at SPC-01. The filtrated supernatant during the first phase of operation is directed to storage tanks for subsequent processing, whereas after the first phase of liquid reduction, the liquid is recirculated for temperature and composition control and RNA derived compound extraction.

The operation may thus include the following steps:
1. Continuously charging the biomass suspension, at a 0.1-30 % wet solid content and eliminating most of the liquid to provide a wet cake. During this operation the biomass suspension consists of viable cells, therefore, the conditions are kept aerobic by regulating the oxygen composition in the centrifuge by the gas control system. This is achieved by either recirculating an oxygen vector or regulating the atmospheric oxygen composition. The filtered liquid is directed to a reservoir or directly to a recirculation loop for reuse in a perfusion type process.
2. When the centrifuge bowl is saturated with the solid material, the liquid stream is recirculated and heated to achieve the RNA degradation temperature. Temperature control is achieved either by microwave heating of the material inside the centrifuge or by recirculation of the liquid controlling the temperature through a heat exchanger (THE-01) and then circulating the said liquid to the bowl of the centrifuge (SPC-01).
3. Once the process temperature is achieved during the inactivation stage, the composition of the atmosphere in the filtration system is switched to a poor oxygen one, for example to an oxygen concentration between 0 and 20% relative to air saturation (0.04 atm of O₂ partial pressure) to ensure low oxidation of the products and reduce by-product formation. This can be achieved by using a nitrogen enriching filter or using pure gases and mixing them to obtain the desired composition. In the case of using oxygen vectors such as perfluorodecalin, the change in the composition is performed by modifying the amount of oxygen in a separated reservoir through gas sparging (nitrogen to lower oxygen, oxygen to increase).
4. Recirculation of liquid can be continued until the process is complete and the liquid diverted to a reservoir for further processing. This liquid is rich in soluble proteins, peptides, amino acids, RNA derivatives, sugars, lipids and fine solids, while at a proper concentration to ensure further cost-effective processing.
5. The liquid material stream is directed to a reservoir tank and kept at a desired temperature and gas atmosphere. For example, where oxygen susceptible material is dissolved, the oxygen can be removed by replacement with a nitrogen enriched atmosphere to ensure conservation.
6. The solid material retained within the centrifuge bowl is treated with liquid to provide a desired composition for further processing. The liquid can be the supernatant, or, in some embodiments, can be a buffer solution. The further processing refers to the next steps to which the biomass is meant to be subjected. For example, immediately after the treatment, separation of the free liquid, thereafter the kneading, forming, and freezing. The goal of the method is to reduce RNA and to make sure that the RNA is removed from the biomass, liquid is required as a vehicle for removal of RNA degradation products to leave the material.
7. Once washed, and the liquid drained, the solid is peeled from the bowl and directed to a screw conveyor to discharge to a proper container or device.

The use of the proposed device reduces the need of several devices for RNA reduction and increases the efficiency by eliminating the need for second heat treatment.

This process can be performed with a syphon shaft peeler centrifuge in batch operation requiring approximately 30 min per batch.

### Example 1b. Application in RNA reduction using a tangential filtration device

Figure 3 shows the equipment for processing cell suspensions using a tangential filtration device with gas diffusion component for oxygen control within the filter. The equipment allows partial liquid removal or culture processing within a filtration device (TFF-101) where temperature, gas composition and filtration/perfusion are controlled. Cells or the biomass can be either recirculated to the production tank or directed to a storage tank (not shown). The filtrate can be either stored in TK-107 or filtered through TFF-102 to perform a particular separation or concentration or just recirculation to ensure fouling reduction in the membrane of TFF-101 by back wash. The gas composition and temperature in the TFF-101 is controlled to ensure the conditions to enhance productivity and accomplish the desired process, for example, for the product modification or maintaining cell viability. TMFCs (Thermal mass flow controllers) control the gas composition to maintain the desired gas levels in the filtration device to ensure proper processing. Gas composition within the filter is attained by interface contact. Alternatively, this interface can be kept controlled by liquid substances containing the gas components (i.e. oxygen vectors for oxygen). Filtration through the TFF-102 can be avoided and liquid stream directed for backwashing the TFF-101. Temperature control can be enhanced by recirculation using heat exchanger THE-01, to ensure direct contact of temperature conditioned liquid with the filter surface.

In one embodiment, gas-liquid transfer (for example, nitrogen, oxygen, or air infusion directly into the liquid) by bubbling gas can be used for gas infusion/diffusion control, particularly during the filtration process. In case of using bubbling gas for a proper control of the gas composition, gas flow rate can be adjusted due to different factors, particularly due to the bubble size. For example, gas flow rate can be 0.1 - 2 VVM (volume of gas relative to volume of the liquid medium per minute) when the bubble size is 3 mm.

The biomass suspended in the culture medium enters the filter (TFF-101) and the biomass is retained within the lumen, due to the filter cut-off. The material is either recirculated to the production tank by using the PS-122 stream or continues to further downstream processing. If necessary, the circulation direction can be reversed (creating a countercurrent) to eliminate obstruction due to material built up in the filter, which is generally observed in the initial portion of the filter. The filter flow can also be inverted from a luminal to transluminal direction (or from a transluminal to luminal direction) to favor the dislodging of retained material. The pumps and valves involved in this flow inversion operations are not shown. TV-01 is a three-way valve that can allow the mixing of recirculated product with the material from the production tank resulting in a concentration and/or temperature conditioning prior to entering to TFF-101. TFF-101 is a mixed-type filter allowing the filtration of the material but also the diffusion of a particular gas composition into the liquid material. The filtrate from TFF-101 is transferred to tank TK-101 for extra filtration in TFF-102 or recirculation by the bypass activated by pump PV-105. The TFF-102 ensures the removal of small molecules and ions, retaining proteins and bigger molecular weight products in the biomass extract retained within the TFF-101. The recirculation involves the passage through a heat exchanger (THE-101) to adjust the temperature of the recirculating liquid to ensure a proper temperature during heat treatment of the biomass retained at TFF-101. The filtered supernatant from the first phase of operation (concentration) is directed to storage tanks for subsequent processing, for extra processing, whereas after the first phase of liquid reduction, the liquid is recirculated for temperature and composition control, and RNA derived compound extraction. Once heat treatment is completed the material is subjected to diafiltration to remove the compound dissolved in the liquid from the biomass or transferred to a separation device for liquid-solid separation to obtain a solid cake.

### Example 1c. Application in RNA reduction using a DSM type filter

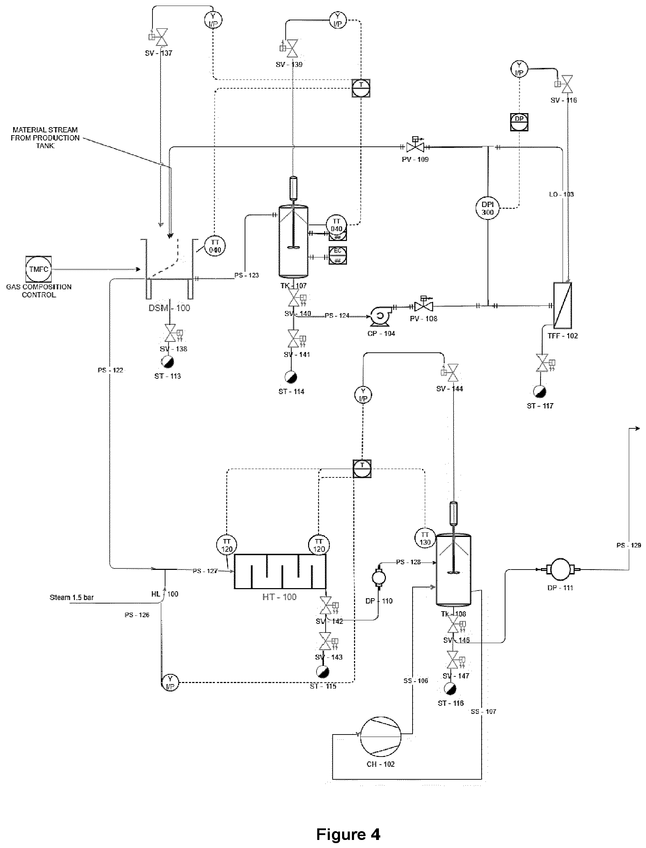

Figure 4 is a schematic diagram of an exemplary system for RNA reduction and biomass processing configuration using a DSM type filter with gas composition control for oxygen control within the filter. The biomass suspended in the culture medium enters the filter (DSM-100) and the biomass is concentrated, and oxygen supplied to avoid hypoxia. Then, the concentrated suspension of biomass is directed to HL-100 for steam infusion for heat control and then kept at RNA degradation temperature for RNA reduction within the holding tank HT-100. Then, it is transferred to the TK-108 where further heat treatment if required takes place. The filtrated from DSM-100 is directed to tank TK-107 and filtered by the filter TFF-102 for recirculation in the filter or liquid recycling in the process. Once heat treatment is completed the material is subjected to final solid liquid separation to obtain the biomass and the biomass extract ready to be dried or used as is

### Example 2. Application in perfusion process of bioreactors

Perfusion in a continuous bioreactor with biomass (total or partial) retention using filtering sleeves or tangential flow filtration apparatuses is affected by the CO₂ built up and O₂ reduction in the filtering device, furthermore, the change in the temperature impacts cell productivity. The control of the gas composition and temperature within the filtering devices with gas phase or gas vector control strategies in this invention greatly improves the performance of perfusion cultures outperforming currently available perfusion systems (See Fig. 3). For tangential-flow-filtration-based devices (e.g., TFF-101 or TFF-102), the filtering device is either a mixed fiber cartridge (for hollow fiber) or a mixed layer cassette, being particular fibers or layers within the device aimed for gas composition and/or temperature controlling function in order to ensure conformation of a filtering device with tight control of the conditions. For example, silicone fibers allow the O₂ transfer, fluorinated polymer fibers are used for heat control applications and regular filtering material fibers for perfusion purposes.

The arrangement of the fibers and composition can be varied in order to suit the particular requirements of the cell being treated.

### Example 3. Use of a static filter for partial dewatering for heat treatment improvement and recirculation.

The liquid stream from the bioreactor is separated in a filtering device allowing the retention of most of the solid material and obtaining a filtrate that can be circulated back to the bioreactor for water saving and nutrient recycling. The filtrate may or may not be processed depending on the quality in terms of contamination control of the filtering device. For example, in Figure 4, the filtering device is indicated as DSM-100. The material with partial liquid reduction named PS-122 is directed to a heat treatment device by either direct steam injection or heat exchange. The biomass coming from the bioreactor is rejected in the filter, and then concentrated. Recirculation of the liquid for multiple times ensures a more efficient filtration as the effective filter cut off size of the filter is reduced due to bed formation from material retained on the filtering surface, increasing the retention capacity expected for the particular screen used (i.e., the filter retains smaller particles and the yield on filtrable solids is increased). In the first case, the condensation of steam results in increased volume and high temperature zones. In the case of heat labile materials, a heat exchanger is preferred, including, but not limited to cask and tube type. As the temperature-controlled processes require a particular residence time, the material is kept at temperature in piston flow or similar equipment allowing control of reaction or treatment time on the product.

The extra concentration in the liquid stream resulting from DSM-100 and the proper control of the gas composition within this operation allow for reduction of the energy required for subsequent separation of the compounds derived from heat treatment of the PS-122 stream. This sensitive reduction of energy consumption renders recovery of the compounds obtained from this stream possible, changing the economy of processes involving heat treatment for proper conditioning of filtrate materials (for example unicellular protein, filamentous fungi biomass, animal derived products with high RNA content). Upon treatment, the material stream (PS-124) is directed to further processing, for example solid-liquid separation to obtain liquid-free heat-treated material (i.e. RNA reduced biomass able to be consumed).

The various embodiments described above are provided by way of illustration only and should not be construed to limit the scope of the disclosure. Various modifications and changes may be made to the principles described herein without following the example embodiments and applications illustrated and described herein, and without departing from the spirit and scope of the disclosure.

## Claims

1. A filtration method for downstream processing of a suspension containing a labile solid material in a liquid medium, comprising controlling a dissolved gas content in the liquid medium during filtering to optimize process conditions.

2. The method according to claim 1, wherein the dissolved gas content is controlled by a gas-liquid interphase transfer process by bubbling gas with a composition and a rate to provide a desired gas content.

3. The method according to claim 1, wherein the dissolved gas content is controlled by a liquid-liquid interphase transfer process by using a gas vector substance charged with a concentration of gas to provide a desired gas content.

4. The method according to claim 1, wherein the dissolved gas content is controlled by a liquid-solid interphase transfer process by using a gas permeable solid with a concentration and pressure of gas by circulation of gas or a gas vector substance within a luminal structure to provide a desired gas content.

5. The method according to claim 1, wherein the dissolved gas content is controlled by a reaction evolving oxygen or other gas/gases.

6. The method according to claim 1, wherein the solid material is removed in a batch operation.

7. The method according to claim 1, wherein the solid material is removed in a continuous operation.

8. The method according to claim 1, wherein the solid material retained during filtering is removed in a mixed-type operation.

9. The method according to claim 1, further comprising the steps of concentrating the suspension by the filtration, heat-treatment, and removal of at least a part of a liquid filtrate from the filtration.

10. The method according to claim 9, further comprising, after removal of the liquid filtrate, heat-treatment and subsequent removal of additional liquid produced by the heat-treatment by further filtration.

11. The method according to claim 9, wherein the solid material is a biomass and the heat treatment inactivates the biomass.

12. The method according to claim 9, wherein the heat treatment comprises treatment with steam, a heated gas, a heated liquid, or combinations thereof.

13. The method according to claim 9, wherein the heat treatment comprises treatment with microwaves.

14. The method according to claim 1, wherein the solid material is a fungus.

15. The method of claim 14, wherein the fungus is a yeast or a filamentous fungus.

16. The method according to claim 1, wherein the solid material is cells of animal origin.

17. The method according to claim 1, wherein the solid material is cells of plant or algal origin.

18. The method according to claim 1, wherein the solid material is cells of protist origin.

19. The method according to claim 1, wherein the solid material is a virus or a part thereof.

20. The method according to claim 1, wherein the solid material is a mixture of two or more of a fungus, cells of animal origin, cells of plant origin, and cells of algal origin.

21. The method according to claim 1, wherein the solid material is present in a colloid, or an emulsified solid or a mixture thereof.
